# EUROPEAN PATENT SPECIFICATION

(11) **EP 1 443 988 B1**
(45) Date of publication and mention of the grant of the patent: **12.09.2007**
(21) Application number: 02749263.6
(22) Date of filing: 16.07.2002
(51) Int. Cl.: A61M 3/02, A61M 35/00, A61K 38/48, C12N 5/06, A61B 17/54, A61B 10/00

(54) **DEVICE FOR CONTROLLED ENZYMATIC REMOVAL AND RETRIEVAL OF TISSUE**
VORRICHTUNG ZUR KONTROLLIERTEN ENZYMATISCHEN ENTFERNUNG UND WIEDERGEWINNUNG VON GEWEBE
DISPOSITIF PERMETTANT L'ELIMINATION ET LA RECUPERATION ENZYMATIQUES COMMANDEES DE TISSU

(30) Priority: 27.07.2001 US 915518
(43) Date of publication of application: 11.08.2004
(73) Proprietor: Ramot at Tel Aviv University Ltd., 69975 Tel Aviv (IL)
(72) Inventor: FREEMAN, Amihay, 73 112 Ben Shemen (IL)
(74) Representative: Schmitz, Jean-Marie
(86) International application number: PCT/IL2002/000572
(87) International publication number: WO 2003/011369

(56) References cited:
- US-A- 3 910 266
- US-A- 5 037 431
- US-A- 5 976 556
- US-A- 6 017 531
- US-B1- 6 264 666

## Description

### FIELD AND BACKGROUND OF THE INVENTION

The present invention relates to a method, device and pharmaceutical composition for the controlled removal of cells from the surface of viable tissue by continuous local application of a solution containing a proteolytic enzyme and, more particularly, to a method, device and pharmaceutical composition for non-surgical, enzymatic treatment and biopsy of skin lesions. The closest prior art is defined by documents US-B1-6264666, US-A-5037431 and US-A-3910266, each of which discloses the features of the preamble of claim 1.

Tissues are composed of individual cells and cell groups, embedded in a proteinaceous extracellular matrix. Collagen fibers are the main component of this ubiquitous network, with other proteins such as fibronectin, laminin, elastin and tenascin, providing a mechanism for cell attachment. Cell surface attachment molecules, such as the CAM proteins, allow cells to adhere to the extracellular matrix and to neighboring cells. Thus, the histological integrity of tissues depends on the interaction of many protein and protein-derived molecules.

Enzymes capable of digesting proteins, or proteases, are commonly employed to disrupt the extracellular matrix of tissues or tissue samples in order to separate cells for establishment of primary cell cultures (1). Proteases used in the isolation of cells for culturing are typically selective in their proteolytic activity or in the method of their application, achieving effective disruption of the matrix and adhesion proteins, yet causing minimal digestion of critical cell components. In the preparation of a primary culture, the tissue is mechanically cut into small (2-3 mm) pieces, these explants washed and gently agitated in an isotonic buffered solution containing a protease, such as trypsin or collagenase, for 30 minutes to several hours at room temperature. This procedure, resulting in suspended, isolated cells, is universal and has been employed for the preparation and propagation of primary cell cultures from a variety of tissues, including skin biopsies (2).

Proteolytic digestion of skin, achieving complete or partial disruption of the tissue, has been applied, typically as an alternative to mechanical means, in a wide variety of industrial, cosmetic, experimental and clinical processes. These include the depilation of animal hides and pelts (3), soothing and promotion of healing of skin lesions such as CO₂ laser surgery wounds (4) and decubitus ulcers (5), the debridement of non-viable tissue as in bum eschar (6), removal of fibrinous exudate from sensitive regions, such as the eye (7), renewal of aging skin by exfoliation (U.S. Patent No. 5,976,556 to Norton, et al), removal of lice nits from hair (U.S. Patent No. 5,935,572 to Sorenson, et al) and the treatment of infectious lesions of the skin such as acne and leprosy (U.S. Patent No. 5,958,406 to de Faire, et al).

### Treatment of skin lesions

Treatment of skin lesions such as lentigines, melasmas, keratoses, nevi, keloids, hypertrophic scars, psoriasis, and tattoos requires the removal of diseased or abnormal skin cells. Surgical procedures are generally painful and destructive to the healthy, neighboring tissues, resulting in scarring and abnormal pigmentation of the treated areas (8), the need for administration of anesthesia, and significant stress trauma to the patient (9). In addition, surgical excision of certain lesions is often complicated by the presence of more than one aberrant cell type (10), and is inappropriate for sensitive and precarious anatomical regions. These disadvantages of surgical excision of skin lesions have led to the development of non-mechanical methods such as electrocauterization, electro-ablation, cryosurgery with liquid nitrogen and lasers.

One currently widely used technique employs laser energy directed at the skin lesion to cause ablation of the undesired tissue. Laser surgery, as it is known, is less traumatic to adjoining tissue, due to the cauterization effects of the intense energy, and the ability to carefully focus the laser beam (11), producing less pain and scarring than scalpel or razor blade excision techniques. However, there remain the problems of pain and scarring associated with the intense heat required for tissue ablation, poor results with attempts at laser treatment of certain lesions, such as melanocytic and congenital nevi (11) and the importance of removal, rather than ablation of the abnormal tissue for histological analysis to determine the character and extent of the lesion.

### Proteolytic enzymes in treatment of skin lesions

U.S. Patents No. 4,226,854 to Klein, et al; 5,505,943 and 6,017,531 to Fortney, et al; 5,106,621 to Rowan, et al and 5,840,283 to Sorenson, et al teach the use of proteases to achieve removal or permeation of abnormal, devitalized or necrotic skin. Rowan, et al. (U.S. Patent No. 5,106,621) disclose a cysteine protease from pineapple, ananain, in a pharmaceutical preparation for topical application and debridement of bum wounds or ulcerated tissue. Similarly, Fortney et al. (U.S. Patents No. 5,505,943 and 6,017,531) describe the use of the bacterial protease Vibriolysin for debridement of bum eschar and necrotized skin by topical application in a solution or ointment preparation. Sorenson et al. (U.S. Patent No. 5,840,283) describe the topical use of proteolytic enzymes as permeation facilitators in treatment of diseased nail, claw or hoof tissue. The commercially available ointment Travase^{™} (U.S. Patent No. 3,409,719 to Noe et al.) also employs proteolytic activity, found in Bacillus subtilis filtrate, for the debridement of bum eschar and decubitus ulcer tissue. In all these, and other similar methods, the proteolytic activity is directed at the removal of non-vitalized tissue and is achieved by individual topical applications with gauze or sponge, with no provision for the control of levels or duration of enzyme activity, or the collection of cells from the treated lesion.

U.S. Patent No. 5,958,406 to de Faire et al. describes the use of a crustacean multifunctional protease for treatment and prevention of bacterial, fungal and viral infections, blood clots, cell-adhesion-related disease (such as HIV and auto-immune disorders) and skin lesions and infection (such as acne, pruritis and scars). The protease preparation is administered by various methods: topically, in an aqueous or non-aqueous vehicle; parenterally, orally or in suppositories for systemic applications; ocularly, in drops, ointment or aerosol; and in cutaneous or sub-cutaneous injection, for skin lesions such as scars, acne and boils. However, no mention is made of control of enzyme activity once applied, or of a means of obtaining cells from the treated tissues.

U.S. Patent No. 5,976,556 to Norton et al. describes the topical application of proteolytic enzymes for exfoliation of skin, and treatment of abnormal conditions and diseases of the skin such as warts, lentigines, melasmas, acne, psoriasis, etc. Control of enzyme activity is effected by the restriction of enzymes to acid proteases, active in their acidic buffer when applied, and inactivated by slow deacidification caused by the normal epidermal pH regulatory mechanism. No ongoing monitoring or control of enzymatic activity is provided, and no mention is made of obtaining cells from the treated tissue.

U.S. Patent No. 6,146,626 to Markert at al. describes the preparation of a proteolytic enzyme mixture comprising collagenase and elastase from *Clostridium histolyticum,* for application in wound healing and obtaining cells from whole tissue or tissue fragments. Conditions for the topical application of the enzyme to bum wounds, and the isolation of cells from a variety of human and other animal tissue are discussed. However, the procedure described relates to preparation of cells for tissue culture from tissue fragments rather than the therapeutic application of cell removal from live tissue. Furthermore, no provision is made for collection of cells from living tissue *in situ.*

### Autolysis of proteases

Proteolytic enzymes, being proteins, are in themselves substrates for self-digestion, or autolysis, thus limiting the effectiveness of active, protease based preparations. For example, a commercially available serine protease derived from bacteria of genus Bacillus (Subtilisin A, manufactured by Novo Nordisk Bioindustry Japan K.K.) loses its enzymatic activity by about 70 %, when it is kept in an aqueous solution at pH 7.0 at 25 °C for 24 hours. Clinical applications employing proteolytic enzymes should provide means of preventing and controlling catalytic inactivation due to autolysis.

Most proteases demonstrate catalytic activity within a defined, and often narrow physico-chemical environment (pH, temperature, ionic strength, solvent polarity, etc.). The specific nature of some proteases may be exploited to prevent autolysis during storage and application of the enzyme, and to allow for the enzymes deactivation after use.

One approach is to store the enzyme in a lyophilized state, to be diluted in an activating buffer of appropriate pH, ionic strength etc. just before or at the time of delivery to the tissue(s) being treated. Enzyme stability is enhanced when dry, but solubility and even dispersal of the enzyme solid in the activating buffer is difficult, resulting in poor control of enzyme activity at point of delivery.

Another method for prolonging and controlling enzyme activity is the separation of enzyme preparations from their activating buffers until use. This separation may be effected physically, storing the enzyme and activating diluent in separate compartments, the enzyme maintained in a stabilized preparation. U.S. Patent No. 6,228,323 to Asgharian et al. describes a device for storage and delivery of proteolytic enzyme preparations intended for dispensing contact lens cleanser. The enzyme preparations are stabilized by polyols, such as PEG-400, in a concentrated form, and are mixed with the activating diluents, in predetermined ratios, upon dispensing. In U.S. Patent No. 5,409,546 to Nakagawa et al. a metal chelator is mixed with the stock enzyme preparation, removing the cations necessary for catalytic activity and prolonging shelf life. Introduction of cations in the diluent restores enzymatic activity, also intended for the cleansing of contact lenses.

A similar approach to prevention of autolysis and destabilization of enzyme preparations is described in U.S. Patent No. 6,117,433 to Edens et al. The effect of polyols on enzyme activity is discussed in detail, as are other enzyme-stabilizing methods such as non-optimal pH, high salt concentrations, etc. The stabilized enzymes, or other biologically active substances, are intended for dispensing with an appropriate amount of activating diluent, for topical application, as in cosmetic preparations, on skin or other external surfaces. No mention is made of removal and retention of cells from treated skin lesions, or of an apparatus for controlled application of a protease solution to a defined and isolated area.

There is thus a widely recognized need for, and it would be highly advantageous to have, a method of controlled enzymatic removal and retention of cells from external surfaces of the skin, devoid of the above limitations.

### SUMMARY OF THE INVENTION

According to the present invention there is provided a device as defined in claim 1 for streaming a solution containing an effective amount of at least one protease, over, and in contact with, a skin portion, the device comprising a first reservoir for containing the solution containing the effective amount of the at least one protease; and an applicator being in fluid communication with the first reservoir and being designed and constructed so as to restrict the streaming of the solution containing the effective amount of the at least one protease, over, and in contact with, the skin portion.

According to further features in the preferred embodiments of the invention described below the device further comprises a second reservoir for containing the at least one protease in a first solution in which the at least one protease in substantially catalytically inactive; and a third reservoir for containing a second solution, the second solution is selected so as to catalytically activate the at least one protease upon mixing with the first solution; the second reservoir and the first reservoir being in fluid communication with the third reservoir.

According to yet further features in the preferred embodiments of the invention described below the activating solution is selected so as to catalytically activate the at least one protease upon mixing therewith.

According to an additional aspect of the present invention there is provided a device for streaming a solution containing an effective amount of at least one protease, over, and in contact with, a skin portion, the device comprising a receptacle for receiving a reservoir containing the solution containing the effective amount of the at least one protease; and an applicator being in fluid communication with the receptacle reservoir when received by the receptacle and being designed and constructed so as to restrict the streaming of the solution containing the effective amount of the at least one protease, over, and in contact with, the skin portion.

According to yet an additional aspect of the present invention there is provided a device for streaming a solution containing an effective amount of at least one protease, over, and in contact with, a skin portion, the device comprising a first receptacle for receiving a first reservoir containing the at least one protease in a first solution in which the at least one protease is substantially catalytically inactive; a second receptacle for receiving a second reservoir containing a second solution, the second solution is selected so as to catalytically activate the at least one protease upon mixing with the first solution; a mixing chamber being in fluid communication with the first and second reservoirs when being received by the first and second receptacles, the mixing chamber being for mixing the first and second solutions so as to obtain the solution containing the effective amount of the at least one protease; and an applicator being in fluid communication with the mixing chamber and being designed and constructed so as to restrict the streaming of the solution containing the effective amount of the at least one protease, over, and in contact with, the skin portion.

According to still an additional aspect of the present invention there is provided a device for streaming a solution containing an effective amount of at least one protease, over, and in contact with, a skin portion, the device comprising a first receptacle for receiving a first reservoir containing the at least one protease; a second receptacle for receiving a second reservoir holding a solution selected so as to catalytically activate the at least one protease upon mixing with the at least one protease; a mechanism for mixing the at least one protease with the solution so as to obtain the solution containing the effective amount of the at least one protease; and an applicator being designed and constructed so as to restrict the streaming of the solution containing the effective amount of the at least one protease, over, and in contact with, the skin portion.

According to yet further features in the preferred embodiments of the invention described below the device comprises a pump for streaming the effective amount of at least one protease from the first reservoir when received by the receptacle to the applicator.

According to still further features in the preferred embodiments of the invention described below streaming the effective amount of at least one protease from the first reservoir to the applicator is effected by gravitation.

According to yet further features in the preferred embodiments of the invention described below the device further comprises a thermoregulator for heating and/or cooling the solution containing the effective amount of the at least one protease.

According to yet further features in the preferred embodiments of the invention described below the device further comprises a mixer for mixing the solution containing the effective amount of the at least one protease.

According to still further features in the preferred embodiments of the invention described below the device further comprises a filter for filtering the solution containing the effective amount of the at least one protease.

According to yet further features in the preferred embodiments of the invention described below the device further comprises a cell collector being in fluid communication with the applicator.

According to further features in the preferred embodiments of the invention described below the cell collector comprises a filter for collecting the cells.

According to still further features in the preferred embodiments of the invention described below the cell collector comprises a continuous flow centrifuge for collecting the cells.

According to further features in the preferred embodiments of the invention described below the applicator comprises a housing having a skin-facing opening, at least one inlet and at least one outlet, the at least one inlet and at least one outlet serve for streaming therethrough and over a skin portion defined by the skin-facing opening, the solution containing the effective amount of the at least one protease.

The present invention successfully addresses the shortcomings of the presently known configurations by providing a device for controlled, non-surgical removal and retrieval of cells from skin lesions. A synergistic effect of proteolytic digestion of the intracellular matrix and "stripping" flow is achieved by treating a defined area with a controlled, continuous stream of proteolytic enzyme solution, causing gentle but effective tissue erosion. Isolated cells from the skin lesion may be collected from the protease solution stream for histological analysis and/or cell culture, affording a method of "enzymatic biopsy". The protease solution may be supplemented with anesthetics, coagulants, anticoagulants and antibiotics to decrease the discomfort, erythema, bleeding, risk of infection and scarring traditionally associated with surgical treatment of skin lesions. Furthermore, delivery of precise levels of catalytic activity may be ensured by controlled activation of stable, inactivated enzyme stock solutions and powders shortly prior to application.

### BRIEF DESCRIPTION OF THE DRAWINGS

The invention is herein described, by way of example only, with reference to the accompanying drawings. With specific reference now to the drawings in detail, it is stressed that the particulars shown are by way of example and for purposes of illustrative discussion of the preferred embodiments of the present invention only, and are presented in the cause of providing what is believed to be the most useful and readily understood description of the principles and conceptual aspects of the invention. In this regard, no attempt is made to show structural details of the invention in more detail than is necessary for a fundamental understanding of the invention, the description taken with the drawings making apparent to those skilled in the art how the several forms of the invention may be embodied in practice.

In the drawings:
FIG. 1 is a cross-sectional view of a device in accordance with one embodiment of the present invention;
FIG. 2 is a cross-sectional view of a device in accordance with another embodiment of the present invention;
FIG. 3 is a cross sectional view of a device in accordance with yet another embodiment of the present invention;
FIG. 4 is a cross sectional view of a device in accordance with sill another embodiment of the present invention;
FIG. 5 is a cross sectional view of a device in accordance with an additional embodiment of the present invention;
FIG. 6 is a cross sectional view of a device in accordance with yet an additional embodiment of the present invention;
FIG. 7 is a cross sectional view of a device in accordance with still an additional embodiment of the present invention;
FIG. 8 is an enlarged, cross-sectional view of the protease solution applicator and engaging mechanism according to the present invention;
FIG. 9 is an enlarged, bottom (skin-facing surface) view of the protease solution applicator, including the protease solution inlet and outlet ports, according to the present invention;
FIG. 10 is a cross sectional view of a device in accordance with the teachings of the present invention which was used while practicing the present invention on skin; and
FIGs. 11 A-B are photographs of histological section of untreated pig skin and pig skin treated with a stream of proteolytic enzymes using the applicator device of Figure 10, according to the present invention.

### DESCRIPTION OF THE PREFERRED EMBODIMENTS

The present inventions concerns devices for the continuous topical application of a solution containing a proteolytic enzyme. Specifically, the present invention can be used for the controlled removal and retrieval of cells from the surface of the skin. Most specifically, the present invention can be used for non-surgical, enzymatic treatment and biopsy of skin lesions such as lentigines, melasmas, keratoses, nevi, keloids, hypertrophic scars, psoriasis and tattoos.

The principles and operation of devices for the controlled removal and retrieval of cells from the surface of the skin according to the present invention may be better understood with reference to the drawings and accompanying descriptions.

Before explaining at least one embodiment of the invention in detail, it is to be understood that the invention is not limited in its application to the details of construction and the arrangement of the components set forth in the following description or illustrated in the drawings. The invention is capable of other embodiments or of being practiced or carried out in various ways. Also, it is to be understood that the phraseology and terminology employed herein is for the purpose of description and should not be regarded as limiting.

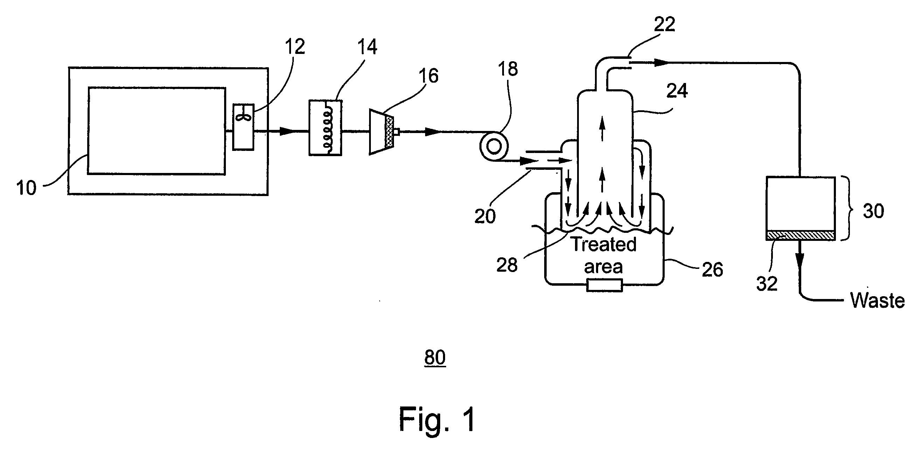

As shown in Figures 1- 9, according to the present invention there is provided a device for streaming and collecting a solution containing an effective amount of protease over, and in contact with, a skin portion, herein referred to below as device **80.**

One preferred embodiment of device **80** is illustrated in Figure 1. Device **80** according to this embodiment, comprises a first reservoir **10** for holding a solution containing an effective amount of at least one protease. First reservoir **10** may be constructed of durable, inert, non-porous material for repetitive uses, such as glass, metal or plastic. First reservoir **10** may be sanitized between uses by methods well known to one skilled in the art, including by moist or dry heat, or the use of antiseptics, gas or radiation. In another preferred embodiment, first reservoir **10** is constructed of non-durable, disposable material such as metal foil, plastic or foil-laminated or impregnated cardboard or paper, for single use, sterilized and sealed for storage. Dimensions of first reservoir **10** may be adequate for containing a volume of protease solution sufficient to complete a single enzymatic surgery procedure, or smaller, necessitating replenishment during the procedure. First reservoir **10** is typically about one liter in volume, but may vary from 100 milliliters to several liters.

In a preferred embodiment, a mixer **12** for mixing the protease solution is in fluid communication with first reservoir **10,** for preventing inconsistent distribution of the protease solution ingredients. Mixer **12** may be external to first reservoir **10,** or indwelling. Mixing may be accomplished by rotary motion, as of an impeller or vane within a chamber, or by a rocking or turning oscillatory motion, as of a rocking or rotating platform.

In another preferred embodiment, first reservoir **10** is in fluid communication with a thermoregulator **14,** for heating and/or cooling the protease solution to optimal temperature for activation of catalytic activity. Thermoregulator **14** may be a radiantly or convection-heated open chamber, receiving the stream of protease solution, or, preferably a heated and/or cooled fluid bath or solid block receiving a fluid communication element, such as glass or plastic tubing, eliminating direct fluid contact of the stream of protease with thermoregulator **14** and reducing risk of contamination of the protease solution with undesired contaminants.

As used herein the phrase "in fluid communication" refers mainly to the capability of selective or non-selective transfer of fluid and/or semi-fluid substances between the specified elements. Such transfer may be accomplished by, for example, channels, tubes, membranes, conduits, pores and/or capillaries.

In yet a further embodiment of the present invention, first reservoir **10** is in fluid communication with a filter **16** which serves for sterilization of the protease solution prior to its application. Filter **16** is preferably a sealed (except for inlet and outlet ports), sterilized housing containing a filtering member excluding particles greater than, for example, 0.25 microns, eliminating common bacterial contamination. One such commercially available filter is distributed under the name Complete Sterifil System (Sigma Chemical Company, Inc.). In a further embodiment of the present invention, first reservoir **10** is in fluid communication with a pump **18** which serves for streaming the protease solution from first reservoir **10** to an applicator **24** (which is described in greater detail hereinafter) under positive pressure. Thus, the protease solution is delivered to the site of treatment with sufficient force to effect a mechanical, "stripping" action in addition to the enzymatic digestion of matrix proteins. The novel combination of a directional, mechanical force and enzymatic disruption of the lesion tissue provided by the present invention enables the removal of cells and tissue from the treated surfaces. Pump **18** may be an air pump, a piston-driven fluid pump, syringe pump or an impeller. In one embodiment of the present invention, pump **18** is preferably a variable-speed peristaltic pump, operating through pressure on a flexible fluid communication element, eliminating direct fluid contact with the protease solution and subsequent risk of contamination. The variable speed feature further affords control of the intensity of the stream of protease solution applied to the dermatological lesion. One such commercially available peristaltic pump is distributed under the name Masterflex Economy (Aldrich Chemical Company, Inc.). In an alternative embodiment of the present invention, streaming the protease solution is effected by gravitation assisted by elevating first reservoir **10** substantially above other elements in fluid communication therewith.

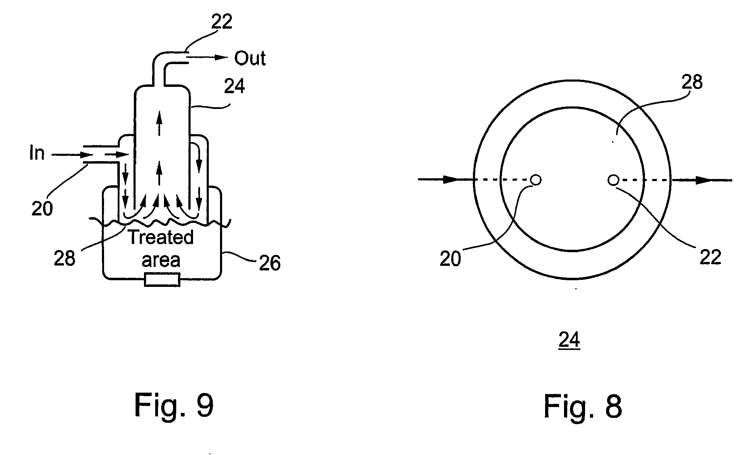

Applicator **24** is in fluid communication with first reservoir **10,** and is designed and constructed to restrict the stream of the protease solution over, and in contact with the skin portion undergoing treatment. Applicator **24** comprises two ports, inlet port **20** serves for receiving the protease solution from first reservoir **10,** and outlet port **22** which serves for removing the protease solution and cells from the treated dermatological lesion. Applicator **24** further comprises a recessed skin-facing surface **28,** enclosed by the downward projecting outer rim of applicator **24,** creating a confined, local area of treatment, preventing exposure of neighboring tissue to proteolytic activity. One presently preferred embodiment of applicator **24** is illustrated in Figures 8 and 9. Inlet port **20** and outlet port **22** provide directional fluid motion for the stream of protease solution, enabling a mechanical "stripping" effect enhancing the enzymatic disruption of the intracellular matrix and removal of cells from the treated lesion surface. Applicator **24** may be engaged with the skin surface by skin-ward pressure applied by attendant operators or treated subject, weight, adhesive connection to adjacent skin surfaces or other means, suitable for the body part bearing the lesion to be treated. In one preferred embodiment applicator **24** comprises an engaging mechanism **26,** which comprises two or more flexible elements adjustably connected to allow encirclement of a cylindrical body part (such as a limb or torso) and application of skin-ward pressure through tension, such as a strap and buckle or toothed belt fastener.

Applicator **24** may be constructed of durable, non-porous material including, but not limited to, glass, metal, plastic or rubber, and may be reuseable or preferably disposable. Applicator **24** is preferably capable of sterilization by gas, chemicals, moist or dry heat, or radiation, and is supplied sealed and sterilized for use. In one alternative embodiment, applicator **24** is a "push-pull" cannula typically employed in tissue perfusion techniques [see, for example, Arancibia, S. (1987) "Push-pull" perfusion technic in neuroendocrinology. Ann Endocrinol (Paris) 48:5; 410-18] which comprises an inflow tube recessed within a wider, outflow tube, creating localized flow of protease solution confined to the outer diameter of the wider, outflow tube.

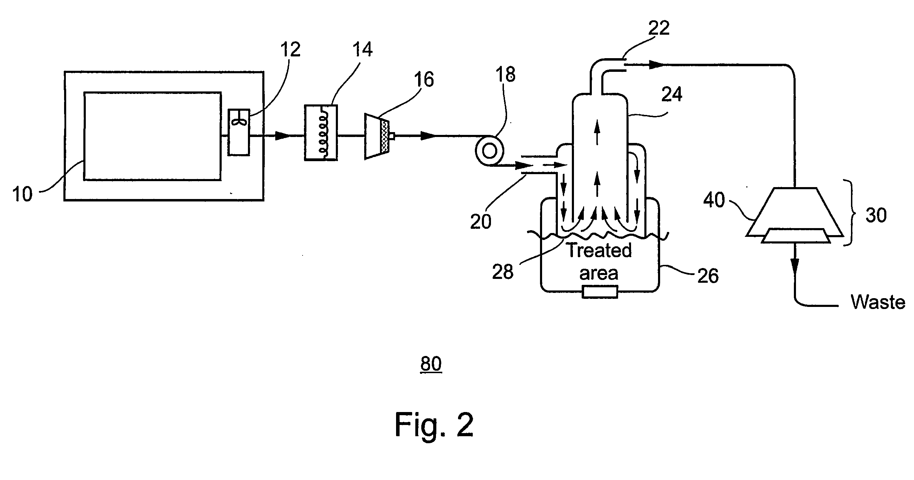

According to the present invention device **80** preferably further comprises a cell collector **30** which is in fluid communication with first reservoir **10** and applicator **24,** and which serves for receiving the protease solution and cells removed from the treated lesion surface, and for providing outflow of waste fluid or fluid to be recycled through device **80.** Collected cells are thus made available for histological examination and/or cell culture procedures. In one preferred embodiment cell collector **30** comprises a filter **32** for collection and separation of cells removed from the dermatological lesion. Collector **30** and filter **32** are preferably supplied as a sterile, disposable modular element, such as the Complete Sterifil System (Sigma, Israel). In a further embodiment of the present invention, which is specifically illustrated in Figure 2, separation of the fluid and cellular fractions in cell collector **30** is effected by continuous flow centrifuge **40.** Continuous flow centrifugation provides increased liquid handling capacity, removing the protease solution outflow quickly upon arrival from applicator **24** and concentrating lesion cells for examination and/or culturing.

It will be appreciated, in the context of the present invention that lesion cells collected by cell collector **30** are exposed to protease activity during separation from the fluid component of the protease stream arriving at cell collector **30.** Preservation of the cells' morphological and metabolic integrity, and therefore diagnostic value, may depend, in part, on limitation of their prolonged contact with protease. Thus, in one preferred embodiment of the present invention, cell collector **30** is constructed to allow removal and/or sampling of collected cells in mid-process. This may be effected by periodic cessation of streaming of protease solution through applicator **24,** removal of the filter element of filter **32,** and replacement with a fresh filter element. Alternatively, the entire cell collector **30** may be replaced during operation with a fresh cell collector unit. Where continuous flow centrifuge **40** is the means of cell collection, centrifuge operation may be periodically halted to allow removal of the collected cells from the centrifuge rotor. More preferably, the centrifuge will provide a continuous outflow of concentrated cells for examination and/or cell culture.

It will be noted that the fluid outflow from cell collector **30** contains largely still active protease solution, devoid of the cellular and tissue debris fractions removed by filter **32** and/or centrifuge **40** which may be recycled for reuse. Thus, in one preferred embodiment the fluid outflow of cell collector **30** is reintroduced to the stream of at least one protease solution "upstream" of applicator **24** and pump **18.** Fluid communication between the cell collector outflow and the stream of protease solution may be effected by a one-way valve connection, ensuring uni-directional streaming of fluid towards applicator **24.** Thus, significant economy of operation is achieved by reuse of the cell collector **30** outflow, effectively reducing the volume of protease solution required per treatment.

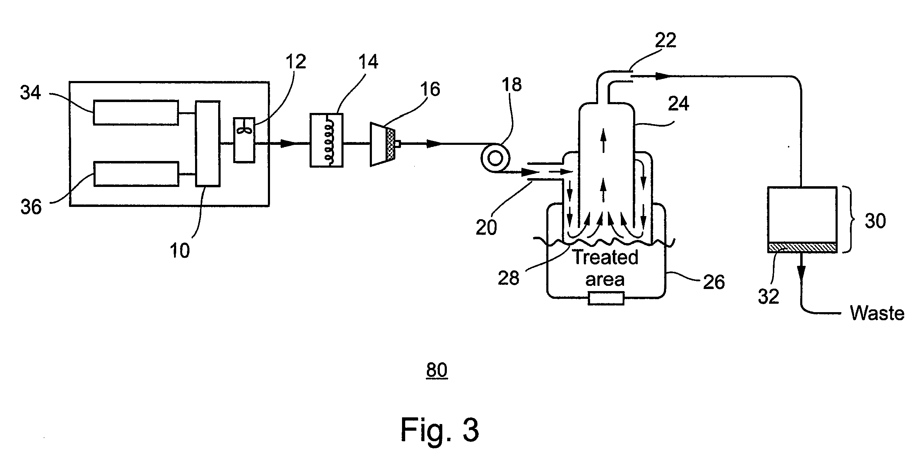

Additional embodiments of enzymatic surgery device **80** are depicted in Figures 3-7; in each case thermoregulator **14,** filter **16,** pump **18,** applicator **24** and cell collector **30** are substantially as described in the preceeding sections.

In one embodiment, illustrated in Figure 3, device **80** comprises, in addition to first reservoir **10,** a second reservoir **34** and a third reservoir **36,** which serve for containing a first, substantially inactive protease solution and a second, protease activating solution, respectively. Thus, the protease solution may be prepared and stored in a stabilized, inactive form prior to use, acquiring substantial catalytic activity only after admixing with the activating solution in first reservoir **10.**

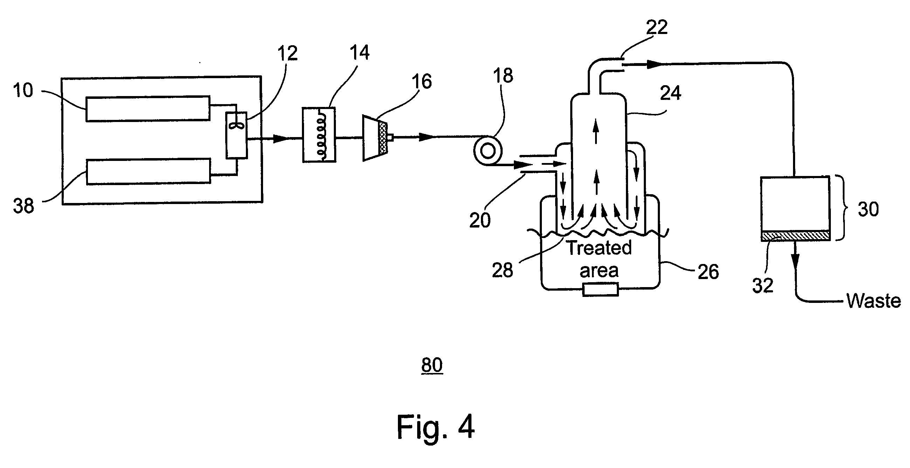

In yet a further embodiment, illustrated in Figure 4, enzymatic surgery device **80** comprises first reservoir **10** and second reservoir **38,** for containing a first, substantially inactive protease and a second, activating solution, respectively. Thus, powdered, lyophylized, and/or other, non-aqueous, stabilized protease preparation(s) placed in first reservoir **10** may be stored until use, minimizing autolysis and loss of catalytic activity. First **10** and second **38** reservoirs are in fluid communication, providing a catalytically active protease solution upon mixing of their contents by mixer **12.**

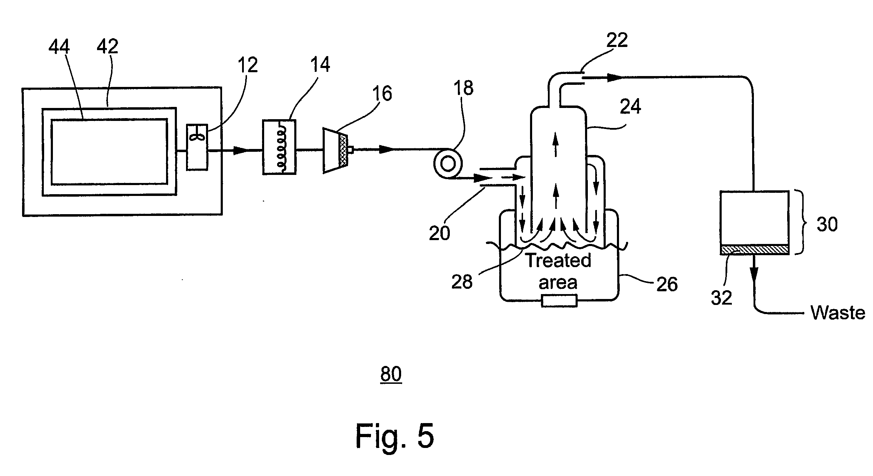

Figures 5-7 depict enzymatic surgery device **80** designed to receive prepared reservoirs or ampoules of protease, protease solution and/or protease activating solution. In one embodiment, illustrated in Figure 5, a receptacle **42** is designed to receive modular reservoir or ampoule **44,** containing catalytically active protease solution, effecting fluid communication with applicator **24,** cell collector **30** and additional "downstream" elements of device **80.** Thus, device **80** may be operated with standardized, pre-prepared, stored protease solution(s), increasing simplicity of use and accuracy of protease activity delivered, and decreasing risk of contamination of treated skin surfaces.

As used herein in the specification and in the claims section below, the terms "reservoir" and "ampoule" interchangeably refer to a separate, enclosed container capable of establishing fluid communication with other containers, receptacles or devices. Such reservoirs or ampoules typically contain fluids or fluid-like substances, and may be designed to be accurately engaged by a complementary receptacle or housing. Sealed reservoirs or ampoules provide convenient, standardized means of preparation and storage of active solutions and reagents for the operation of, for example, enzymatic surgery device **80.**

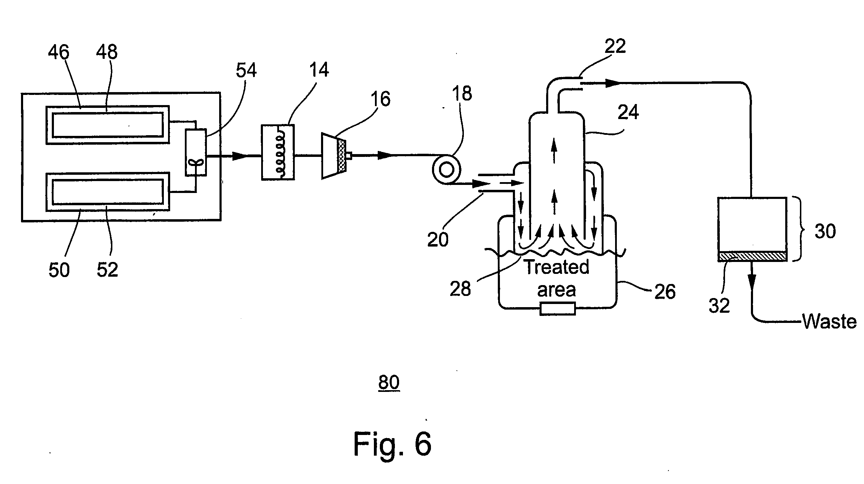

In yet another embodiment, illustrated in Figure 6, first receptacle **46** receives first modular reservoir or ampoule **48,** which contains inactivated, stabilized protease solution, while second receptacle **50** receives second modular reservoir or ampoule **52,** which contains a protease activating solution. First receptacle **46** and second receptacle **50** are in fluid communication with a mixing chamber **54,** which serves for providing fluid contact and mixing of the contents of first reservoir **48** and second reservoir **52,** activating the stabilized, inactivated protease. A mixer **12** as described above can be placed within mixing chamber **54.**

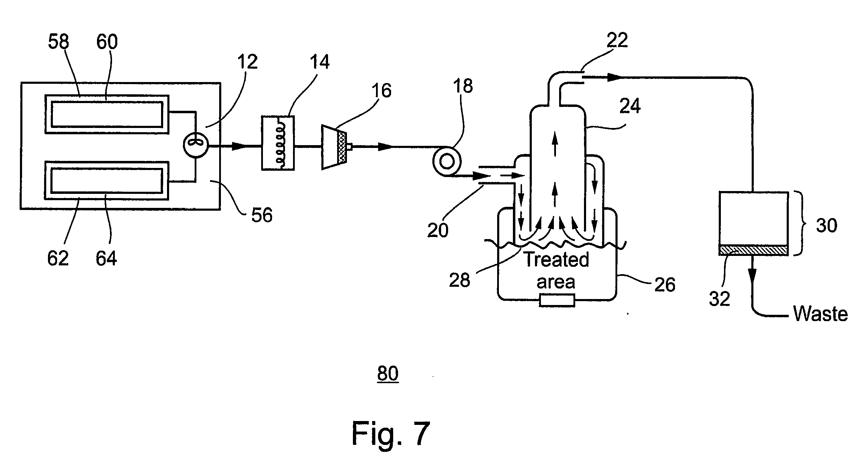

In another embodiment, illustrated in Figure 7, first receptacle **58** receives first modular reservoir or ampoule **60,** which contains stabilized, inactive, protease preparation in powder, lyophilized and/or other non-aqueous form. Second receptacle **62** receives second modular reservoir or ampoule **64,** which contains the activating solution. First receptacle **58** and second receptacle **62** are in fluid communication with mixing mechanism **56,** providing contact between and effect dispersal of the non-aqueous protease preparation in the activating solution.

The ability of proteases to gently disrupt the integrity of dermal tissue has led to the therapeutic use of proteolytic enzymes as an adjunct, or alternative to mechanical or laser surgical treatment of skin lesions. In order for such enzymatic treatment to overcome the abovementioned disadvantages of surgical, electrosurgical, cryosurgical and laser-surgical methods (pain, scarring, traumatic stress, hyperpigmentation and destruction of neighboring tissue), it is desirable for the proteolytic method to readily and thoroughly hydrolyze a wide variety of proteins found in skin lesions; function at physiological pH and temperature; be compatible with adjunct therapies (e.g., anesthetics, cleansing agents, topical antibiotics); and not interfere with normal wound healing or complicate skin grafting. In addition, it is important to provide means of retention and preservation of the viability of the isolated, removed cells for histological examination or cell culture; to allow for localized and confined application of the protease and provide for stability of the enzyme formulations from the effects of pH, temperature and autoproteolysis. These and other beneficial considerations are addressed, for the first time in an integrative approach, by the present invention. Thus, benefits provided by the present invention include gentle enzymatic tissue removal enhanced by mechanical "stripping" action of the locally directed protease stream, superior pain reduction and wound healing provided by inclusion of anesthetics, coagulants/anticoagulants and antibiotics in the protease solution and availability of removed skin cells for histological examination and/or cell culture from the treated lesions. In addition, control of temperature, pH and flow rate of the stream of protease solution, and provision for on-site activation of stabilized enzyme preparations ensure delivery of accurate, effective levels of catalytic activity to the lesion surface.

Proteases are widely applied in the debridement of non-viable tissue (6) and conditioning of skin damaged by CO₂ laser surgery (4) and aging (U.S. Patent No. 5,976,556 to Norton, et al.), exploiting the enzyme's ability to digest protein components of extracellular matrix without damaging healthy tissue. The choice of suitable enzyme preparations, methods of application, and extent of treatment have emphasized the removal of debris and non-viable tissue. Since collagen, elastin, fibrinin and proteoglycan predominate in the skin's extracellular matrix, and are of even greater significance in abnormal conditions such as keloids, scars, warts and fibroses, enzymes of the type collagenase, elastase and hyaluronidase, and combinations thereof, have been most often employed for treatment of dermatological lesions. However, the methods of treatment with these enzymes have been limited to topical application and intradermal injection.

Thus, Pinnell (U.S. Patent No. 4,645,668) teaches the treatment and prevention of acne and hypertrophic scars, keloids, wrinkles and cellulite with repeated intradermal injections of proteases, principally collagenase, with additional hyaluronidase. The author achieved significant resolution of most of the lesions treated, indicating the efficacy of protease digestion of matrix tissue, and reported few, if any, negative effects. However, repeated intradermal injections, over a period of weeks, were required to achieve the desired effects. In addition to the discomfort and protracted character of such a treatment regimen, no retention of cells from the lesions is made possible, necessitating conventional, surgical biopsy methods prior to enzymatic treatment. Similarly, de Faire et al. (U.S. Patent No. 5,958,406) teach the treatment of a variety of conditions associated with cell-adhesion related processes with multifunctional enzyme krill protease, comprising chymotrypsin, trypsin, elastase, collagenase and exo-peptidase activity. Treatment of dermal and internal lesions is addressed, by topical, parenteral, aerosol, systemic, intramuscular and intradermal delivery of the protease compositions. Intradermal injection of proteases is recommended for treatments of scar and keloid lesions. Thus, cell collection or retention from the treated area is not possible and, as in other dermatological enzyme treatment protocols, no control of protease activity after administration is afforded.

Topical application, or injection of proteases offers little control over the level of catalytic activity remaining *in situ,* with autoproteolytic and normal dermal lytic and acidic processes causing unpredictable degradation. Although many protocols for topical or intradermal delivery of proteases depend on individual, empirical results for determining duration of treatment, it has been suggested that topical treatment application of acid proteases, compatible with the normal pH of human skin, can ensure greater control over active enzyme dosage (U.S. Patent 5,976,556 to Norton, et al). However, in the aforementioned invention, as with other topical protease applications, there remains no ongoing control of enzyme activity post treatment.

A method of removing cells from a skin portion of a subject inflicted with a dermatological lesion may be effected by streaming a solution containing an effective amount of at least one protease, over, and in contact with, the skin portion, thereby removing the cells from the skin portion of the subject. By combining enzymatic digestion of intracellular matrix proteins and mechanical disruption of the lesion surface by a fluid force, cells of the treated skin portion become dislodged and may be removed by the stream of at least one protease solution. Non-enzymatic debridement, employing a topical preparation comprising tannic acid and aloe vera has been proposed for treatment of lesions such as keratoses, freckles, dermal ulcers, papilloma, blemishes and benign nevi (U.S. Patent No. 5,420,114 to Clodman, et al). Furthermore, application of the protease solution via streaming onto the lesion surface affords precise localization and control of magnitude and duration of enzymatic activity, through manipulation of enzyme concentrations, pH, temperature, hydrophobicity/hydrophilicity of the enzyme solutions, intensity of streaming, duration and site of contact with protease solution throughout treatment. As described above, enzymatic surgery device 80 of the present invention provides such diverse control of protease treatment through, for example mixer 12, thermoregulator 14, pump 18 and applicator 24.

As used herein, the term "protease" refers to any biologically active molecule, typically a polypeptide, possessing enzymatic peptide hydrolase activity, including endopeptidase and/or exopeptidase activity.

The protease is, but not limited to, vibriolysin, krill protease, chymotrypsin, trypsin, collagenase, elastase, dipase, proteinase K, *Clostridium* multifunctional protease and *Bacillus subtilis* protease. These represent proteases commonly employed in therapeutic methods, have demonstrated low incidence of undesirable side effects, and are commercially available in pure, purified or genetically engineered form [Esperase, Subtilisin A, Savinase, Durazyme (Novo Nordisk Bioindustry Japan K.K.), Protease N "Amano", Protease S "Amano" (Amano Pharmaceutical K.K.), Bioprase (Nagase Seikagaku Kogyo K.K.) and Purified Collagenase (Advance Biofactures, Lynbrook, NY)]. *Clostridium* multifunctional protease and krill protease are easily prepared by one skilled in the art (U.S. Patents Nos. 6,416,626 to Markert, et al, and 5,958,406 to de Faire, et al, respectively). Other proteases which may be selected are papain, bromelain, plasminogen activator, plasmin, mast cell protease, lysosomal hydrolase, streptokinase, pepsin, and any or all fungal, bacterial, plant or animal proteases. The protease solution may contain a single protease, or, preferably, a plurality of proteases. The protease solution may also contain one or more glycosaminoglycans degrading enzyme, such as, but not limited to, various lysosomal hydrolases which include certain endoglycosidases (heparanase and CTAP degrade heparan sulfate and to a lesser extent heparin, and hyaluronidase from sheep or bovine testes degrade hyaluronic acid and chondroitin sulfate), various exoglycosidases (e.g., β-glucoronidase), and sulfatases (iduronate sulfatase), generally acting in sequence to degrade the various glucosaminoglycans. Bacterial lyases such as heparinase I, II and III from *Flavobacterium heparinum* cleave heparin-like molecules, chondroitinase ABC from *Proteus vulgaris,* AC from *Arthrobacter aurescens* or *Flavobacterium heparinum,* B and C from *Flavobacterium heparinum* degrade chondroitin sulfate.

Conventional mechanical and non-mechanical methods of treating and removal of skin lesions such as razor-blade or scalpel excision, CO₂ laser surgery, cryosurgery, electrocauterization, and electroablation are associated with pain, stress trauma, bleeding, scarring, contamination, hyperpigmentation and disruption of adjacent and underlying tissue. The milder proteolytic digestion of skin lesions and wounds has been shown to provide superior healing of such lesions, with decreased incidence of scarring, bleeding and contamination. Indeed, protease preparations are commonly used to promote healing and reduce the scarring of CO₂ laser surgery wounds (4).

Of even greater advantage, then, is the combination of additional topical, non-protease substances capable of reducing undesirable side effects. Schmitt et al (U.S. Patent No. 4,122,158) teaches the application of a biopolymer comprising protease, antibacterial, antibiotic and antifungal substances for the treatment and prevention of scarring and contamination in bum wounds. Even the mild degrees of bleeding, pain and scarring potentially associated with enzymatic removal of cells from skin lesions can be alleviated by application of suitable substances simultaneously with the protease solution. The enzyme surgery device **80** of the present invention is well suited for delivering solutions containing additional active substances compatible with the protease activity, either through their inclusion in the solution or protease in first reservoir **10,** second reservoir **34** or **38,** third reservoir **36,** reservoir or ampoule **44,** first reservoir or ampoule **48,** second reservoir **52,** first reservoir or ampoule **60,** and/or second reservoir or ampoule **64.** Thus, the protease solution may contain at least one of a local anesthetic, a coagulant and an anticoagulant. The protease solution may also contain an effective amount of an antibiotic.

As used herein, the phrase "local anesthetic" refers to any agent applied within a proscribed region (e.g., not systemically) effecting significant reduction or inhibition of activity of nociceptive substances, receptors and/or neural pathways. Non-limiting examples of commonly used local anesthetic agents are cyclo-oxygenase inhibitors (e. g. ibuprofen, indomethacin and ketorolac), 5-hydroxytryptamine receptor antagonists (e.g. amytryptyline), bradykinine receptor antagonists and histamine receptor antagonists.

As used herein, an "effective amount" of antibiotic is intended to include the amount of antibiotic sufficient to significantly prevent and inhibit at least 50 %, preferably 75 % and most preferably 100 % of microbial growth within a dermatological lesion of the subject being treated, such effective amount determined by one skilled in the art.

Preconditioning of the dermatological lesion surface may provide superior efficiency of subsequent protease treatment. Normal epidermis consists of layers of dead squamous cells which provide an effective mechanical barrier protecting the underlying viable dermal layers. Yu et al (U.S. Patent Nos. 4,105,783 and 4,363,815) describe removal of dead cells from the keratin-rich stratum corneum with keratinolytic, desquamifying agent such as low molecular weight hydroxy or keto acids, and their esters. Such exfoliation of the skin is also achieved by cosmetic preparations containing dermabrasives, emollients, detergents, astringents and skin softeners. Thus, the surface of the lesion may be pretreated by streaming of cleansing, softening, astringent, exfoliating and or dermabrasive agents. Device **80** is well suited for this application, requiring only the provision of a suitable pretreatment solution in first reservoir **10,** second reservoir **34** or **38,** third reservoir **36,** reservoir or ampoule **44,** first reservoir or ampoule **48,** second reservoir **52,** first reservoir or ampoule **60,** and/or second reservoir or ampoule **64.**

It will be appreciated that autolysis and loss of functional enzyme concentration from catalytically active preparations of proteases constitutes a significant disadvantage of therapeutic administration of enzymes in topical, injected and/or other compositions. Active shelf life of the protease is limited, and precise control of enzyme activity at the site of administration is virtually unattainable, once injection or topical application is completed. A number of inventions have proposed the storage of biologically active substances, including enzymes, in contact with substances or under conditions limiting their native activity, effectively inactivation and stabilization, until contacted with substantially adequate amount of activating substance, or conditions sufficient to restore biological activity. For example, Edens, et al (U.S. Patent No. 6,117,433) teach the stabilization of biologically active substances, such as vitamins, enzymes and antibiotics in high concentrations by preparation in water activity lowering agents such as salts, polyols, sequestering agents such as EDTA, phyate or gluconate, or antioxidants such as sulphites, glutathione, cysteine or ascorbic acid. Crystallized compositions of biologically active substances, typically more stable than aqueous preparations, are mixed with viscosifying agents to retard precipitation and ensure homogeneity of the biologically active composition. The disclosure further describes a dispensing system for such stabilized formulations, activating the biologically active substance by dilution with an aqueous composition. U.S. Patent No. 5,409,546 to Nakagawa, et al describes the stabilization of *Vibrio* protease for contact lens cleanser composition by addition of polyols, and the specification of a defined range of temperatures (room temperature to about 58 °C) within which the enzyme retains catalytic activity. Rowan et al (U.S. Patent No. 5,106,621) teaches the restoration of catalytic activity of a plant cysteine protease for treatment of bum wounds by addition of cysteine for regeneration of thiol groups. None of the aforementioned examples, however, relate to the administration of proteases for treatment of living cells, nor provide for ongoing, precise control of the activation of catalytic activity at the site of application.

Thus, the protease may be activated shortly prior to streaming the solution containing the effective amount of the at least one protease, over, and in contact with, the treated skin portion. The method wherein the the protease is activated may be effected by: (a) keeping the protease at a first temperature in which the protease is substantially catalytically inactive and heating and/or cooling the at least one protease to a second temperature in which the at least one protease is catalytically active; and/or (b) providing the protease in a powder form and mixing the powder with a solution in which the protease is catalytically active; and/or (c) providing the protease in a first solution in which the protease is substantially catalytically inactive and mixing the first solution with a second solution so as to achieve a mixed solution in which the protease is catalytically active. The second solution may differ from the first solution with respect to pH, ion concentration, free metal concentration, hydrophilicity and hydrophobicity. For example, Figure 1 depicts enzymatic surgery device **80** in fluid communication with thermoregulator **14,** enabling filling of first reservoir **10** with protease solution at sub-optimal, stabilizing temperatures, restoring catalytic activity by raising the temperature of the protease solution only shortly prior to application at the lesion site. Typically, enzymes are substantially inactivated at temperatures below 10 °C, preferably 4 °C. Activation of enzyme catalytic activity may be accomplished by heating and/or cooling the protease solution to optimal temperature, typically in the range of 30 to 40 °C, preferably 37 °C.

As used herein, the term "hydrophilicity" refers to the polar nature of a solution or compound, indicating its tendency to be attracted to other solutions or compounds exhibiting significant dipole moments. Likewise, the term "hydrophobicity" refers to the non-polar nature of a compound or solution, indicating its tendency to be repelled by and immiscible in other compound or solutions exhibiting significant dipole moments.

As used herein, the term "inactivation" refers to the reversible or irreversible suppression or loss of catalytic activity, for example, inactivation rendering proteolytic enzymes incapable of catalyzing hydrolysis of peptide bonds.

It will be appreciated that many enzymes are designated as acid, neutral or basic, according to the physiological environment to which they are adapted. For example, the digestive enzymes pepsin and chymotrypsin, catalytically active in the acidic environment of stomach, exhibit low (pH 3-5) pH optima. Enzymes active in the environment of the dermis will typically have pH optima closer to the milder, acid mantle of the skin (pH 5.5-6.5). Thus, autolysis of the protease of the present invention may be inhibited prior to application by maintaining the protease at a non-optimal pH, and mixing the enzyme solution with an activating solution effectively achieving optimal pH shortly prior to administration to the treated lesion. Thus, in a device as illustrated in Figures 3 and 6, inactive stabilized protease solutions in second reservoir **34** and/or first reservoir or ampoule **48** may be prepared in non-optimal pH, and the activating solution of third reservoir 36 and/or second reservoir or ampoule **52** restores optimal pH for catalytic activity upon mixing shortly prior to administration to the treated lesion. A pH optimum for catalytic activity may be chosen which approximates the mildly acidic normal pH of mammalian skin. Similarly, protease solutions may be inactivated and stabilized by chelation of catalytically critical metal ions such as Ca⁺⁺ or Mg⁺⁺, with EDTA, for example. Activation may be then achieved by providing a concentration of the critical metal ion in the activating solution sufficient to achieve effective and/or optimal metal ion concentrations after mixing. Alternatively, or additionally, proteases may be stabilized and inactivated by preparation in solutions of reduced water availability, as in high salt and polyol concentrations, for example. Restoration of catalytic activity, shortly prior to streaming of the protease solution at the site of treatment, is accomplished by sufficient aqueous dilution by the activating solution. It should be noted that enzymes extracted from different species (i.e., marine, thermophilic, halophilic, euthermic, mammalian, cryophilic, etc.) often demonstrate widely variable and species specific optima of pH, temperature, metal prosthetic group and ion concentration, and polar interactions (hydrophobicity/hydrophilicity).

Protease may be provided in a non-fluid, powder form, mixing with an activating solution shortly prior to application to achieve catalytic activity. The viability of dried enzyme preparations is well know in the art, and many proteases of excellent grades of purity are commercially available in lyophilized form, for example Proteinase K (Sigma-Aldrich, Israel), Clostridopeptidase A (Sigma-Aldrich) and Elastase (Fluka Chemical Company Inc.). However, powdered, lyophilized or granulated enzyme preparations are often difficult to disperse homogeneously in diluent solutions. Thus, in a devices as illustrated in Figure 4, powdered or lyophilized protease preparation(s) may be held in first reservoir **10**, contacted and mixed to homogeneity with activating solution from second reservoir 38 in mixer 12 shortly prior to delivery at the treatment site. Alternatively, in a device as illustrated in Figure 7, the powdered or lyophilized inactivated protease may be provided in separate reservoir or ampoule **60** and is contacted with, and dispersed in, the activating solution, provided in reservoir or ampoule **64,** by the action of mixing mechanism **56** shortly prior to delivery at the treatment site. Thus, the advantages of stabilized, non-aqueous powdered or lyophilized protease preparations may be realised while avoiding the disadvantages of poor dispersal in diluents and imprecise control of enzyme active at delivery.

It will be appreciate that catalytic activity of enzymes may be modified by activators and inhibitors. One such mode of regulation of enzyme activity is reversible inhibition, effected by the interaction of substrate analogs or regulatory molecules which cause changes in substrate binding and/or enzyme kinetics, effectively reducing catalytic activity [see, for example, Enzymes (Chapter 3), in Molecular Cell Biology 1986: Darnell, J, Lodish, H and Baltimore, D, eds. Scientific American Books, Inc]. Since such reversible inhibition of enzyme activity is concentration dependent, restoration of catalytic activity is achieved by contacting the inhibited enzyme preparation with appropriate volumes of diluent devoid of inhibitors. Thus stabilization of the protease solutions may be effected by the inclusion of an effective amount of reversible enzyme inhibitor(s). Activation of the stabilized protease preparation is effected by dilution with adequate volumes of activating solution devoid of inhibitor/and or inhibitor activity.

Similarly, the device of the present invention provides for precise and accurate control of termination of enzymatic activity at the site of treatment and in the collected cells. Inactivation of protease activity effected by manipulation any of the aforementioned methods (pH, ion concentration, free metal concentration, hydrophilicity/hydrophobicity, water availability and reversible inhibition) may be effected by following protease streaming with application of effective amounts of protease-free solution(s) containing, for example, metal chelators, buffers of non-optimal pH and reversible protease inhibitors.

It will be appreciated that many dermatological lesions contain abnormal skin cells and intracellular matrix. For example, psoriatic plaques are caused by abnormal epithelial cell turnover, the collagen of keloids and hypertrophic scars is characterized by abnormal crosslinking, warts are the result of papovaviral infection of epidermal cells, and various types of often hyperpigmented, hyperplastic cells comprise the many types of nevi (moles), keratoses and lentigines. Whereas proteolytic disruption of the intracellular matrix with subsequent resorption of the non-viable tissue has been the objective of previous enzymatic methods, in the present invention the abnormal cells of dermatological lesions are removed, effecting a superior treatment of these skin conditions. Thus, controlled streaming of protease solution may be administered to treat conditions of the skin surface including, but not limited to warts, lentigines, melasmas, acne, keratoses, nevi, keloids, hypertrophic scars, psoriasis and tattoos.

It will be appreciated that the combination of mechanical "stripping" and enzymatic action of a stream of protease solution on the skin surface is suitable for removal of skin cells and debris for esthetic purposes. Thus, controlled streaming of a protease solution may be used to cosmetically treat esthetically undesirable portions of the skin surface.

The device of the present invention may also be applied for the treatment and/or removal of cells from the surface of tissue within a patient, or of internal tissues temporarily exposed during surgical procedures. Markert et al. (U.S. Patent No. 6,146,626) describe the harvesting of cells for tissue culture from internal organs including liver, spleen, heart and skeletal muscle, connective and nerve tissue, glandular tissue, endothelium and others effected by digestion with *Clostridium* collagenase and elastase enzymes. De Faire et al. (U.S. Patent No. 5,958,406) describe the treatment and prevention of infection in internal organs and body cavities by the injection or application of preparations containing krill multifunctional protease activity.

A method of removing and collecting cells from a surface of a viable tissue may be effected by streaming a solution containing an effective amount of at least one protease, over, and in contact with, the surface, thereby removing cells from the surface of the viable tissue, and collecting the cells. The streaming of protease solution may be applied to the tissue surface via an open surgical incision. Protease irrigation, removal and/or sampling for biopsy of a tissue surface or surfaces may be provided via the abovementioned "push-pull" cannula in a closed, fiber optic-directed surgical procedure. Examples of such procedures are arthroscopy, cystoscopy, endoscopy, cholecystoscopy, laparoscopy, colonoscopy, and myringoscopy.

As used herein, the term "treatment" includes the diminishment or alleviation of at least one symptom associated or caused by the disorder being treated. For example, treatment can be diminishment of several symptoms of a disorder or complete eradication of a disorder.

The importance of obtaining cells from dermatological lesions cannot be overstated. Treatment without determining accurate diagnosis may remove the lesion, but will often incur unnecessary scarring, recurrences, and financial hardships. Of particular importance is the determination of cells type(s) comprising nevi and keratoses, due to the widespread prevalence of these lesions in adults, and their potential for malignant transformation (Sosis, A., Benign Tumors of the Skin, in Skin Diseases: Diagnosis and Management in Clinical Practice (1982), Binnick, S.A. ed, Addison-Wesley Publishing Co., USA. 166-230). As mentioned above, previous methods of non-surgical treatment of skin lesions, such as laser surgery, electrosurgery and chemical or enzymatic ablation have not provided any means for obtaining cells from the lesions, necessitating the use of traditional surgical biopsy techniques for accurate diagnosis.

It will be appreciated that confining the enzymatic activity to a stream of protease solution directed at the lesion surface, rather than topical application of creams or intradermal injection, provides the opportunity for retention of the cells removed from the treated lesion. Thus, a method of removing and collecting cells from a skin portion of a subject inflicted with a dermatological lesion may be effected by streaming a solution containing an effective amount of at least one protease, over, and in contact with, the skin portion, thereby removing the cells from the skin portion of the subject; and collecting the cells. The products of protease digestion at the site of treatment, the site defined by the perimeter of skin-facing surface **28** of applicator **24,** may be removed through outflow port **22,** and are transferred to cell collector **30,** in fluid communication with applicator **24.** Separation of the fluid and cellular components of the outflow of protease solution from applicator **24** may be accomplished by filtration, or, in another embodiment, by continuous flow centrifugation, as described above. Small volume continuous flow centrifuges, commonly used for separation of blood components (for example, the OrthoPAT® System, Haemonetics Corporation, Braintree, MA) are commercially available and are easily adapted to the device of the present invention through fluid communication, as illustrated in Figure 2. Alternatively, cell collection may be effected by retention on a column capable of adsorbing cells through interaction with proteinaceous, poly- and/or oligo saccharide or other cell-surface components.

Known cell separations involve several techniques, some of which are based on specific affinities. Other cell separation techniques rely on more serendipitous mechanisms such as entrapment of target cells in supports of various origins and structures. See, for example, Wigzell and Anderson, J. Exp. Med. 129:23-36, 1969; Rutishauser et al. Proc. Natl. Acad. Sci. 70, 1973; Wysocki and Sato, Proc. Natl. Acad. Sci. 75:2844-2848, 1978; Antoine et al. Immunochem. 15, 1987. See also, U.S. Pat. No. 6,008,040 to Datar. The basic process of affinity separation entails creating contact between cell mixtures to be separated and a support matrix to enable the target cells to preferentially attach, bind, adsorb or become trapped to and within the support, and then washing away the undesired cells, or *vice versa.* Specific affinity techniques use monoclonal antibodies to recognize specific markers on the membranes of cells and to "attract" the target cells to bind to the monoclonal antibodies. Specific affinity "attractions" of target cells also may occur by hydrophobic or hydrophilic interactions, metal-affinities, ion exchangers, and the like. Thus, cell collection may be effected by passage of the outflow stream from applicator **24** through cell collector 30 and contacting with a device, e.g. a cell-binding column, capable of retention of the cells and their separation from the outflow stream.

A composition comprising at least one protease and at least one substance selected from: a local anesthetic, a coagulant and an anti-coagulant may be provided. For example, a pharmaceutical composition may comprise a pharmaceutically acceptable carrier and, as active ingredients, at least one protease and one or more of the following: a local anesthetic, a coagulant and an anti-coagulant. The composition further may contain an effective amount of an antibiotic.

As used herein, the term "coagulant" is defined as any agent that promotes clotting, or coagulation of blood, which may be safely applied to a dermatological lesion. An example of such a coagulant material comprising gelatin, thrombin and calcium is described in U.S. Patent No. 6,045,570 to Epstein, et al. Likewise, the term "anti-coagulant" refers to any agent which retards, inhibits or prevents the clotting or coagulation of blood, which may be safely applied to a dermatogical lesion, such as heparins, coumarins or other agents possessing thrombolytic activity.

The language "effective amount" is intended to include the amount of the at least one protease sufficient to remove or significantly reduce progression of a dermatological lesion of the subject being treated. An effective amount can be determined on an individual basis and will be based, at least in part, on consideration of the severity of the symptoms to be treated and the activity of the specific protease selected. Thus, an effective amount of the at least one protease can be determined by one of ordinary skill in the art employing such factors as described above using no more than routine experimentation in health care management.

As used herein in the specification and in the claims section below, the phrase "pharmaceutically acceptable carrier" refers to a pharmaceutically acceptable material, composition or vehicle, such as a liquid filler, diluent, solvent or encapsulating material, involved in carrying or transporting a compound(s) of the present invention within or to the subject such that it can perform its intended function. Each carrier must be "acceptable" in the sense of being compatible with the other ingredients of the formulation and not injurious to the patient. Some examples of materials which can serve as pharmaceutically acceptable carriers include: sugars, such as lactose, glucose and sucrose; starches, such as corn starch and potato starch; cellulose, and its derivatives, such as sodium carboxymethyl cellulose, ethyl cellulose and cellulose acetate; powdered tragacanth; malt; gelatin; talc; oils, such as peanut oil, cottonseed oil, safflower oil, sesame oil, olive oil, corn oil and soybean oil; glycols, such as propylene glycol; polyols, such as glycerin, sorbitol, mannitol and polyethylene glycol; esters, such as ethyl oleate and ethyl laurate; agar; buffering agents, such as magnesium hydroxide and aluminum hydroxide; alginic acid; fruit acids, pyrogen-free water; isotonic saline; Ringer's solution; ethyl alcohol; phosphate buffer solutions; and other non-toxic compatible substances employed in pharmaceutical formulations.

The pharmaceutical compositions mentioned above may be formulated as solutions typically include a pharmaceutically-acceptable aqueous or organic solvent. The phrases "pharmaceutically-acceptable aqueous solvent" and "pharmaceutically-acceptable organic solvent" refer to a solvent that is capable of having dispersed or dissolved therein the active compound, and possesses acceptable safety properties (e.g., irritation and sensitization characteristics). Water is a typical aqueous solvent. Examples of suitable organic solvents include: propylene glycol, butylene glycol, polyethylene glycol (200-600), polypropylene glycol (425-2025), glycerol, 1,2,4-butanetriol, sorbitol esters, 1,2,-6-hexanetriol, ethanol, isopropanol, butanediol, and mixtures thereof. These solutions may contain from about 0.01 % to about 50% of the active compound, more preferably from about 0.1 % to about 20%; and from about 1% to about 80% of an acceptable aqueous or organic solvent, more preferably from about 1% to about 40%.

Additional objects, advantages, and novel features of the present invention will become apparent to one ordinarily skilled in the art upon examination of the following examples, which are not intended to be limiting. Additionally, each of the various embodiments and aspects of the present invention as delineated hereinabove and as claimed in the claims section below finds experimental support in the following examples.

### EXAMPLES

Reference is now made to the following example, which together with the above descriptions, illustrate the invention in a non limiting fashion.

### EXAMPLE 1

### Enzymatic Epidermis Removal

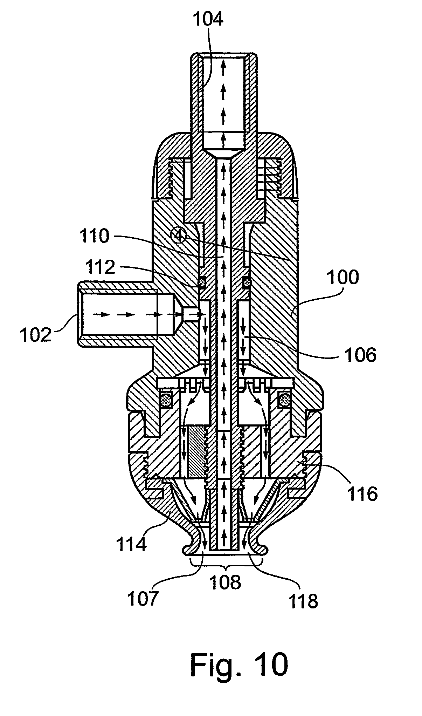

***Design and fabrication of an applicator-device for the treatment of skin surface area 9 mm in diameter:*** An applicator-device for the treatment of skin areas, 9 mm in diameter, was designed as described in Figure 10. The applicator-device was fabricated in a workshop with special emphasis on making the skin contacting surface from silicone to ensure flexibility and effective sealing.

Briefly, with reference now to Figure 10, the applicator-device used in this experiment comprises a housing **100** having an inlet **102** and an outlet **104.** Fluid entering through inlet **102** is directed via a first tube structure **106** to a treatment zone **107** defined by a somewhat conical silicon structure **114** having a skin-facing opening **108,** 9 mm in diameter. A second tube structure **110** positioned within first tube structure **106** is used to direct fluid from treatment zone **107** to outlet **104.** An O-ring **112** is used to restrict flow to the intended direction within first tube structure **106.** A screw mechanism **116** allows adjustment of the height of opening **118** of second tube structure **110** with respect to skin-facing opening **108** of treatment zone **107.** A pump (not shown) is used to direct fluid from a reservoir (not shown) into inlet **102.** A draining tube (not shown) is used to drain fluid from outlet **104.**

***Enzymatic epidermis removal:*** Using the applicator-device of Figure 10, an enzyme solution containing Collagenase (1 mg/ml, Sigma Cat. No. C0130) and Thermolysin (0.5 mg/ml, Sigma type x, Cat. No. P1512) in 0.1 M PBS buffer, pH 7.5, was applied onto a skin sample freshly removed from an adult female large-white (1 year old, 90 kg) pig, mounted on a flat holder and pre-cleaned with 70 %(v/v) aqueous ethanol, at a flow rate of 3-4 ml/hour for 3 hours at room temperature.

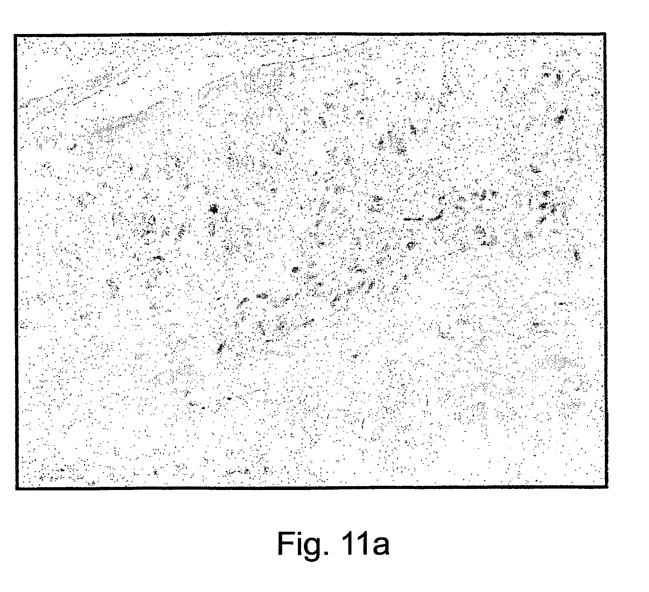

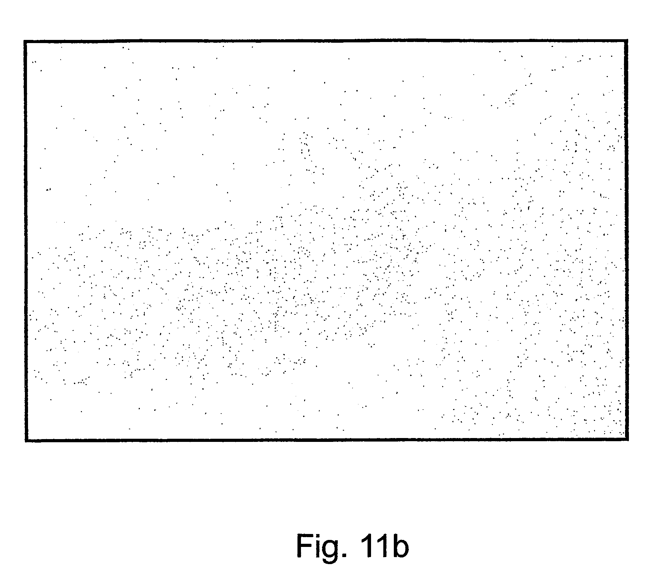

Following this treatment and detouchment of the applicator-device, complete hair removal from the treated area, accompanied by the formation of smooth, crater like removal of skin volume was macroscopically observed. The skin sample was immediately fixed in neutral buffered formalin (4 % v/v) for 48 hours. The skin was then rinsed with distilled water, dehydrated in alcohol and embedded in paraffin. Stained histological serial sections (0.8 µm in thickness) were then prepared in a plane parallel to the Epidermis-Dermis direction, mounted on slides, stained with Hematoxillin-Eosin and examined under light microscope. Examination of the edges of the treated area clearly indicated enzymatic epidermis removal from the treated area (Figures 11B) as compared to untreated skin (Figure 11A).

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination.

Although the invention has been described in conjunction with specific embodiments thereof, it is evident that many alternatives, modifications and variations will be apparent to those skilled in the art. Accordingly, it is intended to embrace all such alternatives, modifications and variations that fall within the scope of the appended claims. In addition, citation or identification of any reference in this application shall not be construed as an admission that such reference is available as prior art to the present invention.

### LIST OF REFERENCES CITED

1. Ferkushny, RI. 1983. In: "Culture of Animal Cells", AR Liss, NY. Pp. 108.
2. Hybbinette, S., Bostrom, M. and Lindberg, K. 1999. Enzymatic disassociation of keratinocytes from human skin biopsies for in vitro cell propagation. Experimental Dermatology, 8, 30-38.
3. DE Patent application 19519436 (28.11.96)
4. Gaspar, L. and Bogdanyi, E. 1998. Clinical experience with enzymes in the treatment of skin lesions caused by CO2 laser surgery. Orv. Hetil. 139, 1475-77 (Hungarian).
5. Spoelhof, G.D. and Ide, K. 1993. Pressure ulcers in nursing home patients. Am. Fam. Physic. 47, 1207-15.
6. Mekkes, J.R., LePoole, I.C., Das, P.K., Bos, J.D. and Westerhof, H. 1998. Efficient debridement of necrotic wounds using proteolytic enzymes derived from Antarctic krill. Wound Repair and Regeneration, 6, 50-57.
7. Mullaney, P.B., Wheeler, D.T. and al-Nahdi, T. 1996. Dissolution of pseudophakic fibrinous exudates with intraocular streptokinase. Eye, 10, 362-66.
8. Gambichler, T., Senger, E., Rapp, S., Almouti, D., Altmeyer, P. and Hoffman, K. 2000. Deep shave excision of macular melanocyte nevi. Dermatol. Surg., July 26 (7), 662-66.
9. Augustin, M., Zschocke, I., Godau, N., Buke-Kirschenbaum, A., Peschen, M., Sommer, B. and Sattler, G. 1999. Skin surgery under local anesthesia leads to stress-induced alterations of psychological, physical and immune functions. Dermat. Surg., Nov 25 (11), 868-71.
10. Crawford, J.B., Howes, E.L. Jr. and Char, D.A. 1999. Conjunctival combined nevi. Trans-Am. Ophthamol. Soc., 97, 170-83.
11. Raulin, C., Schanermark, M.P., Greve, B. and Werner, S. 1995. Q-switched ruby laser treatment of tattoos and benign pigmented skin lesions. A critical review. Ann. Plast. Surg., Nov 41 (5), 555-65.

## Claims

1. A device (80) for removing cells from a skin portion of a subject, comprising:
(a) a first reservoir (10) containing a solution containing an effective amount of at least one protease; and
(b) an applicator (24) in fluid communication with said first reservoir (10), for restricting streaming of said protease solution, over, and in contact with, the skin portion, said applicator (24) including :
(i) an inlet port (20) for receiving said protease solution from said first reservoir;
(ii) a first tube structure operatively connected to said inlet port (20) for directing said streaming of said protease solution from said inlet port (20) to a treatment zone of the skin portion, such that said streaming protease solution enzymatically and mechanically causes the removal of cells from the skin portion;
**characterised by**:
(iii) a second tube structure positioned within said first tube structure for adjustably directing said streaming protease solution and said removed cells away from said treatment zone, wherein a screw mechanism operatively connected to said second tube structure allows adjustment of height of opening of said second tube structure with respect to a skin-facing opening of said treatment zone; and
(iv) an outlet port (22) operatively connected to said second tube structure for removing said streaming protease solution and said removed cells from said second tube structure, thereby removing the cells from the skin portion of the subject.

2. The device of claim 1, further comprising a pump (18) for effecting said streaming of said protease solution from said first reservoir to said applicator (24).

3. The device of claim 1, wherein said streaming of said protease solution from said first reservoir to said applicator (24) is effected by gravitation.

4. The device of claim 1, further comprising a thermoregulator (14) for heating and/or cooling said protease solution.

5. The device of claim 1, further comprising a mixer (12) for mixing said protease solution.

6. The device of claim 1, further comprising a filter (16) for filtering said protease solution.

7. The device of claim 1, further comprising:
a second reservoir (34) containing said at least one protease in a first solution in which said at least one protease is substantially catalytically inactive; and
a third reservoir containing a protease activating solution, said activating solution activates catalytic activity of said at least one protease upon mixing with said first solution;
said second reservoir (34) and said first reservoir (10) are in fluid communication with said third reservoir.

8. The device of claim 1, further comprising a second reservoir (34) for containing a protease activating solution, said activating solution activates catalytic activity of said at least one protease upon mixing therewith.

9. The device of claim 1, further comprising a cell collector (30) in fluid communication with said applicator (24), for receiving said streaming protease solution and said removed cells from said outlet port (22).

10. The device of claim 9, wherein said cell collector (30) comprises a filter (16) for collecting said removed cells from the skin portion of the subject.

11. The device of claim 9, wherein said cell collector (30) comprises a continuous flow centrifuge (40) for collecting said removed cells from the skin portion of the subject.

12. The device of claim 1, further comprising an engaging mechanism (26) for engaging said applicator (24) to the skin portion of the subject.

13. The device of claim 1, further comprising a receptacle for receiving said first reservoir (10).

14. The device of any one of claims 1 to 6 or 8 to 13 wherein:
the first reservoir (48) contains a first solution containing an effective amount of at least one protease in a substantially catalytically inactive form;
the device further comprising a first receptacle (46) for receiving said first reservoir (48);
a second reservoir (52) containing a protease activating solution, said activating solution activates catalytic activity of said at least one protease upon mixing with said first solution;
a second receptacle (50) for receiving said second reservoir (52);
a mixing chamber (54) in fluid communication with said first (48) and second (52) reservoir when received by said first (46) and second (50) receptacles, said mixing chamber being for mixing said first solution and said activating solution such that said at least one protease becomes catalytically active in solution; wherein
the inlet port (20) receives said active protease solution from said mixing chamber.

15. The device of claim 14, wherein the at least one protease being in a non aqueous catalytically inactive form.

## Patentansprüche

1. Vorrichtung (80) zum Entfernen von Zellen von einem Hautbereich eines Subjekts, umfassend:
(a) einen ersten Behälter (10), der eine Lösung enthält, die eine wirksame Menge mindestens einer Protease enthält; und
(b) einen Applikator (24) in Fluidkommunikation mit dem ersten Behälter (10) zur Einschränkung des Fließens der Proteaselösung über den und in Kontakt mit dem Hautbereich, wobei der Applikator (24) umfasst:
(i) eine Einlassöffnung (20) zur Aufnahme der Proteaselösung von dem ersten Behälter;
(ii) eine mit der Einlassöffnung (20) in Wirkverbindung stehende erste Schlauchstruktur zum Lenken des Fließens der Proteaselösung von der Einlassöffnung (20) zu einer Behandlungszone des Hautbereichs, sodass fließende Proteaselösung enzymatisch und mechanisch das Entfernen von Zellen von dem Hautbereich verusacht;
**gekennzeichnet durch**:
(iii) eine in der ersten Schlauchstruktur positionierte zweite Schlauchstruktur zum verstellbaren Lenken der fließenden Proteaselösung und der entfernten Zellen weg von der Behandlungszone, wobei ein mit der zweiten Schlauchstruktur in Wirkverbindung stehender Schraubmechanismus die Einstellung der Höhe der Öffnung der zweiten Schlauchstruktur in Bezug zu einer der Haut zugewandten Öffnung der Behandlungszone gestattet; und
(iv) eine mit der zweiten Schlauchstruktur in Wirkverbindung stehende Auslassöffnung (22) zum Entfernen der fließenden Proteaselösung und der entfernten Zellen aus der zweiten Schlauchstruktur, wodurch die Zellen von dem Hautbereich des Subjekts entfernt werden.

2. Vorrichtung von Anspruch 1, weiter eine Pumpe (18) zum Bewirken des Fließens der Proteaselösung von dem ersten Behälter zu dem Applikator (24) umfassend.

3. Vorrichtung von Anspruch 1, wobei das Fließen der Proteaselösung von dem ersten Behälter zu dem Applikator (24) durch Schwerkraft bewirkt wird.

4. Vorrichtung von Anspruch 1, weiter einen Thermoregler (14) zum Erwärmen und/oder Kühlen der Proteaselösung umfassend.

5. Vorrichtung von Anspruch 1, weiter einen Mischer (12) zum Mischen der Proteaselösung umfassend.

6. Vorrichtung von Anspruch 1, weiter einen Filter (16) zum Filtrieren der Proteaselösung umfassend.

7. Vorrichtung von Anspruch 1, weiter umfassend:
einen zweiten Behälter (34), der die mindestens eine Protease in einer ersten Lösung enthält, worin die mindestens eine Protease im Wesentlichen katalytisch inaktiv ist; und
einen dritten Behälter, der eine Proteaseaktivierungslösung enthält, wobei die Aktivierungslösung die katalytische Aktivität der mindestens einen Protease beim Mischen mit der ersten Lösung aktiviert;
wobei der zweite Behälter (34) und der erste Behälter (10) in Fluidkommunikation mit dem dritten Behälter sind.

8. Vorrichtung von Anspruch 1, weiter einen zweiten Behälter (34) zum Enthalten einer Proteaseaktivierungslösung umfassend, wobei die Aktivierungslösung die katalytische Aktivität der mindestens einen Protease beim Mischen damit aktiviert.

9. Vorrichtung von Anspruch 1, weiter einen Zellsammler (30) in Fluidverbindung mit dem Applikator (24) zur Aufnahme der fließenden Proteaselösung und der entfernten Zellen von der Auslassöffnung (22) umfassend.

10. Vorrichtung von Anspruch 9, wobei der Zellsammler (30) einen Filter (16) zum Sammeln der entfernten Zellen von dem Hautbereich des Subjekts umfasst.

11. Vorrichtung von Anspruch 9, wobei der Zellsammler (30) eine Durchlaufzentrifuge (40) zum Sammeln der entfernten Zellen von dem Hautbereich des Subjekts umfasst.

12. Vorrichtung von Anspruch 1, weiter einen Eingreifmechanismus (26) zum Angreifenlassen des Applikators (24) an dem Hautbereich des Subjekts umfassend.

13. Vorrichtung von Anspruch 1, weiter einen Aufnahmebehälter zur Aufnahme des ersten Behälters (10) umfassend.

14. Vorrichtung von einem der Ansprüche 1 bis 6 oder 8 bis 13, wobei:
der erste Behälter (48) eine erste Lösung enthält, die eine wirksame Menge mindestens einer Protease in einer im Wesentlichen katalytisch inaktiven Form enthält;
wobei die Vorrichtung weiter umfasst:
einen ersten Aufnahmebehälter (46) zur Aufnahme des ersten Behälters (48);
einen zweiten Behälter (52), der eine Proteaseaktivierungslösung enthält, wobei die Aktivierungslösung die katalytische Aktivität der mindestens einen Protease beim Mischen mit der ersten Lösung aktiviert;
einen zweiten Aufnahmebehälter (50) zur Aufnahme des zweiten Behälters (52);
eine Mischkammer (54) in Fluidkommunikation mit dem ersten (48) und zweiten (52) Behälter, wenn sie von dem ersten (46) und dem zweiten (50) Aufnahmebehälter aufgenommen sind, wobei die Mischkammer zum Mischen der ersten Lösung und der Aktivierungslösung dient, sodass die mindestens eine Protease katalytisch aktiv in Lösung wird; wobei
die Einlassöffnung (20) die aktive Proteaselösung von der Mischkammer erhält.

15. Vorrichtung von Anspruch 14, wobei die mindestens eine Protease in einer nicht-wässrigen, katalytisch inaktiven Form vorliegt.

## Revendications

1. Dispositif (80) pour prélever des cellules d'une portion de la peau d'un sujet, comprenant :
(a) un premier réservoir (10) contenant une solution contenant une quantité efficace d'au moins une protéase ; et
(b) un applicateur (23) mis en communication par fluide avec ledit premier réservoir (10) pour restreindre l'écoulement de ladite solution de protéase par-dessus la portion de la peau en contact avec cette dernière, ledit applicateur (24) englobant :
(i) un orifice d'entrée (20) pour recevoir ladite solution de protéase à partir dudit premier réservoir ;
(ii) une première structure tubulaire reliée de manière opérante audit orifice d'entrée (20) pour diriger ledit courant de ladite solution de protéase depuis ledit orifice d'entrée (20) jusqu'à une zone de traitement de la portion de la peau, de telle sorte que ladite solution de protéase qui s'écoule donne lieu à un prélèvement enzymatique et mécanique de cellules à partir de la portion de la peau ;
**caractérisé par** :
(iii) une deuxième structure tubulaire disposée au sein de ladite première structure tubulaire pour diriger de manière réglable ladite solution de protéase qui s'écoule et lesdites cellules prélevées à l'écart de ladite zone de traitement, un mécanisme à vis relié de manière opérante à ladite deuxième structure tubulaire permettant de régler la hauteur d'ouverture de ladite deuxième structure tubulaire par rapport à une ouverture disposée face à la peau de ladite zone de traitement ; et
(iv) un orifice de sortie (22) relié de manière opérante à ladite deuxième structure tubulaire pour retirer de ladite deuxième structure tubulaire ladite solution de protéase qui s'écoule et lesdites cellules prélevées, pour ainsi prélever les cellules de la portion de la peau du sujet.

2. Dispositif selon la revendication 1, comprenant en outre une pompe (18) pour mettre en oeuvre ledit écoulement de ladite solution de protéase à partir dudit premier réservoir jusqu'audit applicateur (24).

3. Dispositif selon la revendication 1, dans lequel ledit écoulement de ladite solution de protéase à partir dudit premier réservoir jusqu'audit applicateur (24) est mis en oeuvre par la force de gravitation.

4. Dispositif selon la revendication 1, comprenant en outre un thermorégulateur (14) pour chauffer et/ou pour refroidir ladite solution de protéase.

5. Dispositif selon la revendication 1, comprenant en outre un mélangeur (12) pour mélanger ladite solution de protéase.

6. Dispositif selon la revendication 1, comprenant en outre un filtre (16) pour filtrer ladite solution de protéase.

7. Dispositif selon la revendication 1, comprenant en outre :
un deuxième réservoir (34) contenant ladite au moins une protéase dans une première solution dans laquelle ladite au moins une protéase est essentiellement inactive du point de vue catalytique ; et
un troisième réservoir contenant une solution d'activation de protéase, ladite solution d'activation activant l'activité catalytique de ladite au moins une protéase lors du mélange de cette dernière avec ladite première solution ;
ledit deuxième réservoir (34) et ledit premier réservoir (10) étant mis en communication par fluide avec ledit troisième réservoir.

8. Dispositif selon la revendication 1, comprenant en outre un deuxième réservoir (34) destiné à contenir une solution d'activation de protéase, ladite solution d'activation activant l'activité catalytique de ladite au moins une protéase lors de son mélange avec cette dernière.

9. Dispositif selon la revendication 1, comprenant en outre un collecteur de cellules (30) mis en communication par fluide avec ledit applicateur (24) pour recevoir ladite solution de protéase qui s'écoule et lesdites cellules prélevées à partir dudit orifice de sortie (22).

10. Dispositif selon la revendication 9, dans lequel ledit collecteur de cellules (30) comprend un filtre (16) pour récolter lesdites cellules prélevées à partir de la portion de la peau du sujet.

11. Dispositif selon la revendication 9, dans lequel ledit collecteur de cellules (30) comprend une centrifugeuse à écoulement continu (40) pour récolter lesdites cellules prélevées à partir de la portion de la peau du sujet.

12. Dispositif selon la revendication 1, comprenant en outre un mécanisme de mise en contact (26) pour la mise en contact dudit applicateur (24) avec la portion de la peau du sujet.

13. Dispositif selon la revendication 1, comprenant en outre un récipient pour la réception dudit premier réservoir (10).

14. Dispositif selon l'une quelconque des revendications 6 ou 8 à 13, dans lequel :
le premier réservoir (48) contient une première solution contenant une quantité efficace d'au moins une protéase sous une forme essentiellement inactive du point de vue catalytique ;
le dispositif comprenant en outre :
un premier récipient (46) pour la réception dudit premier réservoir (48) ;
un deuxième réservoir (52) contenant une solution d'activation de protéase, ladite solution d'activation activant l'activité catalytique de ladite au moins une protéase lors du mélange de cette dernière avec ladite première solution ;
un deuxième récipient (50) pour recevoir ledit deuxième réservoir (52) ;
une chambre de mélange (54) mise en communication par fluide avec ledit premier réservoir (48) et avec ledit deuxième réservoir (52) lors de la réception par ledit premier récipient (46) et par ledit deuxième récipient (50), ladite chambre de mélange étant conçue pour mélanger ladite première solution et ladite solution d'activation de telle sorte que ladite au moins une protéase manifeste une activité catalytique en solution ;
dans lequel l'orifice d'entrée (20) reçoit ladite solution de protéase active à partir de ladite chambre de mélange.

15. Dispositif selon la revendication 14, dans lequel ladite au moins une protéase se trouve dans une forme non aqueuse catalytiquement inactive.
